**Europäisches Patentamt**

(19) **European Patent Office**

**Office européen des brevets**

(11) Publication number : **0 444 120 B1**

(12) **EUROPEAN PATENT SPECIFICATION**

(45) Date of publication of patent specification :
01.09.93 Bulletin 93/35

(51) Int. Cl.$^5$ : **C12Q 1/68**

(21) Application number : **90900191.9**

(22) Date of filing : **21.11.89**

(86) International application number :
**PCT/EP89/01416**

(87) International publication number :
**WO 90/06042 14.06.90 Gazette 37/76**

(54) **DETECTION AND QUANTITATIVE DETERMINATION OF RNA AND DNA.**

(30) Priority : **21.11.88 GB 8827160**

(43) Date of publication of application :
**04.09.91 Bulletin 91/36**

(45) Publication of the grant of the patent :
**01.09.93 Bulletin 93/35**

(84) Designated Contracting States :
**AT BE CH DE ES FR GB IT LI LU NL SE**

(56) References cited :
**EP-A- 0 125 995**
**EP-A- 0 265 244**
**EP-A- 0 278 220**
**EP-A- 0 297 379**
**GB-A- 2 202 328**

(73) Proprietor : **DYNAL A.S.**
**P.O. Box 158 Skoyen**
**N-0212 Oslo 2 (NO)**

(72) Inventor : **HORNES, Erik**
**Lilleakeron 9B**
**N-0283 Oslo 2 (NO)**
Inventor : **KORSNES, Lars**
**Monolittveien 12**
**N-0375 Oslo 3 (NO)**

(74) Representative : **Holmes, Michael John et al**
**Frank B. Dehn & Co. Imperial House 15-19**
**Kingsway**
**London WC2B 6UZ (GB)**

Jouve, 18, rue Saint-Denis, 75001 PARIS

## Description

This invention relates to the detection and quantitative determination of RNA and DNA and to kits for performing such assays.

DNA and RNA may be detected and/or assayed by a number of conventional techniques using solution and/or supported reactions. In general, a specific DNA or RNA sequence is isolated from a sample by electrophoresis or diffusion on to a support such as a nitrocellulose membrane-filter and then a labelled probe, which selectively hybridises only to a target nucleic acid, is added to the support. A common type of probe is single stranded (ss) DNA complementary to a sequence in the target DNA or RNA.

The hybrid molecule so formed may then be detected by a variety of techniques depending on the nature of the label used. An example of such a hybridisation system is US Patent No. 4,358,535 of Falkow et al.

One method of labelling a probe is to incorporate a radioactive atom such as $^{32}P$, $^{14}C$ or $^{3}H$, e.g. by the nick translation method of Rigbny et al (J. Mol. Biol, 113:237, 1977) whereby a labelled nucleotide is incorporated into a gap created in the DNA of the probe. Other labels can be introduced by nick translation, for example by incorporating biotinylated nucleosides which can then be coupled to an avidin bound label such as an enzyme. The DNA can also be labelled with antigenic groups reacting with antibodies.

For assay or quantification of nucleic acids, e.g. DNA or mRNA, either the total nucleic acid material present in a sample or that transcribed from a specific gene, is conventionally determined by the so-called dot-blot analysis technique. In this technique a suitable probe, labelled for example with $^{32}P$ or $^{35}S$ is hybridised onto a membrane filter carrying dots of the sample nucleic acid at varying dilutions together with dots of reference nucleic acid at varying known dilutions. After autoradiography, the intensity of the sample dots may be compared visually or by a densitometer with the reference dots to give an estimate of the concentration of the nucleic acid.

Nucleic acids are often present in quantities which are too small to be assayed by conventional dot blotting and may often advantageously be amplified by the PCR (Polymerase Chain Reaction) technique. In the case of DNA, the unamplified dsDNA is denatured and primers are annealed to both the coding and the non-coding strand. The primers are preferably those corresponding to the 5'-terminal sequences of the DNA so that on extension of the primer with a polymerase, the whole DNA sequence of each strand will be replicated. If, however, the intended assay requires hybridisation of a labelled probe only to a central sequence of the DNA, it will be adequate for the primers to hybridise to sites on either side of that section so that transcription produces copies of shorter DNA sequences including the hybridisation site of the probe. The double stranded DNA so produced is then denatured by raising the temperature followed by rapid cooling. An excess of the primer molecules is present and are annealed to the newly formed coding and non-coding strands. Extension using polymerase produces further double stranded DNA. The temperature cycling can be repeated many times, thereby producing a large number of copies of the DNA. Preferably, the polymerase used is one which can withstand the highest temperature of the cycle, commonly the Taq polymerase, otherwise there is a need to separate the polymerase from the nucleic acids before each heating step or replenish the polymerase after each cooling step. Both the latter options are expensive and add to the complexity of the technique.

Even with the advance made by the use of PCR a worker still needs to fix the target DNA to a support during the step of hybridisation to the labelled probe so that it can be immobilized during washing steps e.g. to remove unbound labelled probe. Usually the fixing is non-specific, especially when using nitrocellulose filters, and one often finds background labelling due to the probe becoming non-specifically fixed to the support.

It has been suggested, e.g. in US 4672040 (Advanced Magnetics) and EP 265244 (Amoco Corp.), that magnetic particles may be used as supports for DNA probes.

There is a need to provide a method for the detection and/or quantitative determination of RNA or DNA which overcomes the difficulties which exist with prior methods. It is an object of the invention to meet this need.

Accordingly, the invention provides a method for the detection and/or quantitative determination of target RNA or DNA in an analyte sample which includes the steps of:

a) contacting the analyte with magnetic particles carrying a single stranded 5'-attached DNA probe capable of binding to said RNA or DNA;

b) contacting the magnetic particles with an aqueous solution of a reverse transcriptase or polymerase in the presence of labelled nucleotides using the probe as a primer whereby labelled complementary single stranded DNA is formed if said RNA or DNA was present in said analyte to serve as template; magnetically aggregating the magnetic particles onto a surface and removing said solution; and

c) detecting the presence or absence of said label and/or determining the amount of said label.

It is one advantage of this invention that labelling takes place using standard labelled nucleotides rather than a labelled probe, the probe in this case serving the triple function of selecting the target nucleic acid, immobilising the latter for ease of separation from unbound label and serving as a primer. This is particularly advantageous when radioactive labels are concerned since it is not necessary for the probe to be labelled as a preliminary step thereby leading to a delay which may reduce the activity of the label by radioactive decay. A further advantage is that the radioactive signal polymerised onto the primer on the beads can be directly analysed on a scintillation counter, avoiding long autoradiograph exposure times. The method is characterised by its simplicity and rapidity as compared to previous techniques of nucleic acid assay.

The term "label" as used herein is intended to encompass both direct labels, such as radioactive elements, fluorophores and chromophores, as well as indirect labels where a low molecular weight ligand of a specific binding pair is attached to a nucleotide and thus serves as an attachment for a label. Examples of such indirect labels and specific binding pairs include biotin, which can be affinity bound by avidin or streptavidin and haptens which can be affinity bound by antibodies or binding fragments thereof. The larger binding partner may not, in itself, be a label but may have a label, such as an enzyme, latex bead, fluorophore or chromophore attached thereto.

Clearly, it would be expensive to label a whole gene or large portion thereof. Accordingly, it is a preferred feature of the invention that the chain length of complementary single stranded DNA is limited. This feature may be achieved, for example, by the addition of some dideoxy nucleotides during the labelling polymerisations to limit the chain length of the labelled cDNA which is synthesised.

Where the sequence of the target nucleic acid is known, it is possible to synthesise a defined cDNA chain by omitting one or more of the labelled nucleotides so that synthesis stops at a known point. It is possible in such cases to use a single labelled nucleotide where this would produce sufficient attached label for detection.

Furthermore the ratio of dideoxy nucleotides to other nucleotides controls the average length of the cDNA chain and thus the average number of labelled bases hybridised to each molecule of target nucleic acid. If the number of labels on each target molecule is known either precisely or as an average, the total signal divided by the signal from one label multiplied by the above known number of labels gives a direct indication of the number of molecules of target nucleic acid.

Another method of introducing a known number of labels is to design the probe to hybridize to a sequence of the target nucleic acid which is spaced from the 5' terminus by a known number of bases. Reaction with labelled nucleotides and a polymerase thus incorporates a precisely known number of labelled bases.

In yet another method of limiting chain length, when dealing with assays for mRNA, one can provide a poly-dT probe of sufficient length to bind polyA "tails" and serve as a primer yet it is shorter than the polyA "tails". After hybridisation, a polymerase and thymidine are added, at least some of the thymidine being labelled, and a complementary labelled polyT cDNA is produced which extends from the end of the primer to the first non-adenyl residue of the mRNA. This qualitative method can be quantitative if one knows the lengths of the poly-dT probe and the polyA "tails" and one determines statistically the average length of cDNA which will be produced. The signals from each cDNA can be readily determined on the basis of the ratio of labelled: non-labelled thymidine.

The method according to the invention can advantageously include PCR amplification of the target nucleic acid between steps (a) and (b). Where the nucleic acid is mRNA, this can be bound to magnetic particles carrying oligo-dT probes and reacted with reverse transcriptase to produce cDNA. The non-coding strand is then synthesised using a polymerase. The dsDNA so produced can then be denatured and the coding and non-coding strands hybridised to specific DNA probes attached to magnetic particles, these probes serving as the primers for PCR amplification using a suitable polymerase. If an excess of the magnetic particles separately carrying the two specific DNA probes/primers is present, to hybridise to the cDNA strands as they are produced, the PCR temperature cycle can be applied and repeated until adequate amplification has been effected. The final step of PCR amplification should be denaturation to produce the single stranded DNA for the labelling reaction.

Particular uses of the method of the invention include:

1. Detection and/or quantification of a specific DNA sequence using magnetic particles with a specific probe, for example in detecting the presence of specific viruses or bacteria in body fluids or contaminated food, or detecting genomic genotypes in human diagnosis, (optionally with PCR or other amplification).

2. Quantification of total cellular mRNA using magnetic particles with an oligo-dT probe, for example in small scale purifications.

3. Detection and/or quantification of a specific mRNA sequence using magnetic particles with a specific probe, for example in studying gene expression, especially levels of gene activity (e.g. oncogene transcription levels in cancer diagnosis) and detection of viruses such as HIV which are integrated in cell genomes or free in the cells.

4. In addition to isolating the target mRNA or DNA having the magnetic particles, this can be directly assayed by incorporating labelled nucleotides into an aliquot of the isolated nucleic acid using the same magnetic probe (and a polymerase or reverse transcriptase) so that the label can provide a direct determination of the target DNA or RNA isolated.

Several advantages of the use of magnetic particles stand out clearly. The magnetic particles can be added to a mixture containing the target nucleic acid, e.g. a cell extract, stirred and then magnetically drawn to one side of the receptacle. The liquid can then be removed together with unwanted components and the magnetic particles, having the RNA bound thereto, can then be redispersed in a washing solution. The washing step can be repeated several times in quick succession. The whole process of obtaining the target nucleic acid can be performed in under 15 minutes.

A further advantage is the ease with which hybridisation or any process effected using the magnetic particles can be continuously monitored by magnetically aggregating the particles at intervals and assaying a label associated either with the material on the particles or with material in the supernatant.

The use of magnetic aggregation to separate the particles is far less vigorous than traditional separation techniques such as centrifugation which generate shear forces which can degrade nucleic acids or proteins.

The use of magnetic aggregation to separate the particles is far less vigorous than traditional separation techniques such as centrifugation and thus high shear forces which can degrade nucleic acids or proteins are avoided. Moreover, the ease with which the magnetic particles can be separated from solution and redispersed in a different solution lends itself especially to automation, in particular in automated synthesis or assay methods.

As indicated above, the probes can be DNA moieties which will hybridise with RNA or DNA. These include oligo-dT, which will hybridise with the poly A 'tails' universally present on native mRNA, and probes comprising specific DNA sequences which hybridise with specific sequences in target DNA and RNA molecules. Each probe may consist of a directly attached single stranded DNA which may be oligo-dT or a specific DNA sequence or it may be attached to the magnetic particle via a double stranded piece of DNA.

Where the method of the invention is applied to the detection and/or quantification of all the mRNA material from a cell lysate, the probe is advantageously oligo-dT, that is a relatively short chain of deoxythymidine units, e.g. from 20 to 200 bases. Such a chain may be readily and cheaply prepared by enzymic polymerisation of deoxythymidine units, e.g. from 20 to 200 bases. Such a chain may be readily and cheaply prepared by enzymic polymerisation of deoxythymidine.

The oligo-dT probes may be directly attached covalently to the beads. It may be advantageous to attach the probe to the bead via a linker sequence comprising a restriction endonuclease (RE) specific site, so that the probe and hybridised DNA or mRNA can, if desired, be liberated into solution by mild enzymatic reaction. In the conventional detection and quantification methods particularly when automated, the enzymic operations are carried out in the same unchanged buffer which is thus not optimised for each reaction. However, using magnetic particles according to the invention allows one to change buffers and the like and thereby optimise the production of cDNA. Further, in the case of ss cDNA synthesis, the ratio of nucleotide reagents to mRNA is usually kept approximately stoichiometric in order to avoid contaminating succeeding stages with excess reagent. The ease and speed of washing, which comes from the use of magnetic particles according to the invention, permits excess reagents to be used, with a consequent increase of efficiency.

To avoid random hybridisation of unwanted nucleic acid and to complete the removal of the remaining components of the hybridisation solution, the magnetic particles are preferably washed at least once after the initial magnetic separation. To remove nucleic acid bound by random partial homology, the washing may be carried out under stringent conditions, either by increasing the temperature or by using a lower salt concentration than that used in hybridisation, e.g. 0.5M sodium chloride or an equivalent solution.

Stringency is normally calculated according to the probe length and G:C content. If the homology between the probe oligonucleotide and the target nucleic acid is inexact, washing should be carried out under less stringent conditions. In general, washing should be carried out at a temperature 12°C below the melting temperature of the duplex ($T_m$). The approximate $T_m$ may be conveniently calculated according to the following relationships (taken from Maniatis, T. et al (1982) Molecular Cloning; a laboratory manual pages 388-389).

(a) $T_m = 69.3 + 0.41 \cdot (G+C)\% - 650/L$  L equals the average length of the probe in nucleotides.

(b) The $T_m$ duplex DNA decreases by 1°C with every increase of 1% in the number of mismatched base pairs.

(c) $(T_m)u_2 - (T_m)u_1 = 18.5 \log_{10}\frac{u_1}{u_2}$

where $u_1$ and $u_2$ are the ionic strengths of two solutions.

For small oligonucleotides, the melt temperature may be approximated in degrees centigrade as follows:

$T_m = 2 \times$ (number of A+T residues) $+ 4 \times$ (number of G+C residues)

The hybridisation reaction is preferably effected in a 1M sodium chloride solution or an equivalent solution known in the art. (See Nucleic Acid Hybridisation, B D Hames and S J Higgins, IRL Press, 1985).

In one embodiment of the invention the probe is oligo-dT and all the mRNA in a cell extract can thus be isolated by hybridisation to the poly A tails present on all native eukaryotic mRNA molecules. A particular advantage of this embodiment is that attachment of the oligo-dT DNA probe to the bead via its 5' terminus, allows the probe to also serve as a primer for reverse transcription not only to incorporate labelled bases in accordance with the invention but, using the same magnetic probe, to synthesize sscDNA. Such synthesis is more fully described in our copending application of even date herewith claiming priority from United Kingdom Patent Application No. 8827158.0.

The target nucleic acid may require an initial treatment step before performance of the method according to the invention. For example, if the target nucleic acid is dsDNA an initial melting step followed by rapid cooling will be necessary so as to provide ssDNA. (Determination of the melt temperature Tm is discussed above). If it is desired to assay a particular mRNA from a pool of mRNA then, due to the lower stability of RNA compared with DNA it may be preferable to first synthesize cDNA for all mRNA species in the pool before selecting the target nucleic acid with the specific probe. If the target nucleic acid is of relatively low abundance then it is preferable to amplify it using PCR if, of course, two spaced specific sequences are known.

It is clear that the initial step of mRNA selection is much simpler in the process according to the invention since a specific probe is used to isolate a particular mRNA and the mRNA size fractionation and identification stages of the prior art are not necessary, thus avoiding lengthy delays and consequent mRNA degradation.

In the conventional process of mRNA isolation and purification, successive enzymic operations are carried out in the same vessel; by products being precipitated at each stage with ethanol and the enzyme being removed by phenol extraction at each stage. This makes it difficult to optimise the conditions for each reaction. In the present method, due to the ease of separation of the magnetic particles from the supernatant, an optimal buffer can be used at each stage.

A particularly useful form of probe for use where subsequent cDNA synthesis is intended is a DNA sequence in which the 3' end overlaps and is hybridised to a region near the 5' end, leaving the remainder of the 5'-terminal region as a sticky end to hybridise with the target nucleic acid. If a functional group such as an amino group is present in a position distal from the sticky end, the loop may be covalently attached to the magnetic particle e.g. via carboxyl groups. Alternatively, a biotin group may be attached to the loop and thus bind the probe to streptavidin coated particles. DNA having a terminal region corresponding to the sticky end will thus have the possibility of being ligated to the adjacent part of the loop if it is required to secure the DNA covalently. RE sites can be provided in the overlap region of the probe for subsequent detachment of the DNA.

The use of specific DNA probes coupled to magnetic particles is of particular value in detection and quantification of families of mRNA molecules having a common sequence hybridising to the probe. Thus, for example, the mRNA coding for immunoglobulin may be assayed from a relevant cell extract using DNA probes from the constant regions of the heavy and light chains.

In the study of genetically transmitted diseases, it is possible to assay mRNA corresponding to a series of modified genes using a probe corresponding to a conserved sequence of the gene and to synthesise the corresponding DNA of the genes using the methods described above.

The probe oligonucleotide may be prepared by using any of the commercially available DNA synthesis devices, e.g. those available from Applied Biosystems, Inc. (850-T Lincoln Center Drive, Foster City, CA 94404).

The particles are particularly advantageously monodisperse and/or superparamagnetic. Both these properties greatly assist the kinetics of reactions in which the particles are involved. It is a surprising feature of the invention that the probes carried by the particles react in the reactions virtually as rapidly as if free in solution. Thus, as mentioned earlier, total isolation of mRNA for example can be effected in about 15 minutes in contrast with the 2 hour period using an affinity column. By using monodisperse particles, that is particles of substantially the same size, the reaction rate and other parameters are particularly uniform. By using superparamagnetic particles (that is particles containing sub-particles of ferromagnetic material which are smaller than the domain size required to maintain permanent magnetism), one can avoid magnetic aggregation or clumping of the particles during reaction, thus again ensuring uniform and rapid reaction kinetics. The particles can be readily aggregated onto a surface by application of a magnetic field and then be readily re-dispersed for a subsequent treatment step, e.g. by physical agitation.

The preferred magnetic particles for use in this invention are monodisperse superparamagnetic beads produced according to EP 83901406.5 (Sintef), the disclosure of which is incorporated herein by reference. In these beads, the iron is very uniformly distributed and provides a very uniform response to a magnetic field which is important in designing a reproducible procedure, particularly for automation, since all the beads move at the same speed. Furthermore, since a reproducible amount of iron can be incorporated in each particle, this can be adjusted to a relatively low level which permits the specific gravity of the particles to be in the range specified below. In the case of prior, less regular products, small particles either had too little iron to counteract Brownian forces when a magnet was applied or the specific gravity of the material led to undesirable sedimentation of the larger particles. Some automated systems use magnetic fields to restrain the particles within a reaction zone while solutions are passed through; uniform magnetic and rheological properties are essential in magnetic particles for use in such a system.

The term "monodisperse" used herein is intended to encompass size dispersions having a diameter standard deviation of less than 5%.

We prefer to use beads having a specific gravity in the range 1.1 to 1.8 most particularly 1.2 to 1.5. In the monodisperse beads used in accordance with the invention, the specific gravity is, again, particularly uniform, leading to uniform and predictable kinetic characteristics.

Advantageously, the monodisperse particles are spherical beads of diameter at least 1 and preferably at least 2 microns, being preferably not more than 10 and more preferably not more than 6 microns in diameter e.g. about 3 microns. Smaller particles sediment more slowly and in some cases the sedimentation time may be long compared to the reaction time, thus avoiding the need for physical agitation. However, particles of mean diameter 0.1 to 1.5 microns including fine particles of much smaller diameter, as used in the prior art, behave unreliably in response to magnetisation.

The attachment of the probes to the particles may be by direct chemical bonding as well as affinity binding, by streptavidin/biotin complexes and the like.

For attachment of the probes, the magnetic particles may carry functional groups such as hydroxyl, carboxyl, aldehyde or amino groups. These may in general be provided by treating uncoated monodisperse, superparamagnetic beads, to provide a surface coating of a polymer carrying one of such functional groups, e.g. polyurethane together with a polyglycol to provide hydroxyl groups, or a cellulose derivative to provide hydroxyl groups, a polymer or copolymer of acrylic acid or methacrylic acid to provide carboxyl groups or an aminoalkylated polymer to provide amino groups. US Patent No. 4654267 describes the introduction of many such surface coatings.

Preferred coated particles for use in the present invention may be prepared by modification of the beads according to the US Patents 4336173, 4459378 and 4654267, the disclosure of which is incorporated herein by reference. Thus, for example, macroreticular porous polymer particles, prepared from styrene-divinylbenzene and with a diameter of 3.15um were treated with $HNO_3$ to and with a diameter of 3.15um were treated with $HNO_3$ to introduce $-NO_2$ groups at the surface of the pores. Then the particles were dispersed in an aqueous solution of $Fe^{2+}$. The $Fe^{2+}$ is oxidised by the $-NO_2$ groups which leads to precipitation of insoluble iron oxy-hydroxy compounds inside the pores. After heating the iron exists as finely divided grains of magnetic iron oxides throughout the volume of the porous particles. The $NO_2$ groups are reduced by the reaction with $Fe^{++}$ to $NH_2$ groups.

To fill up the pores and to introduce the desired functional groups at the surfaces, different monomers are caused to polymerize in the pores and at the surface. In the case of a preferred type of particle, the surface carries -OH groups connected to the polymeric backbone through $-(CH_2CH_2O)_{8-10}$ linkages. Other preferred beads carry -COOH groups obtained through polymerization of methacrylic acid.

Thus, for example, the $NH_2$ groups initially present in the beads may be reacted with a diepoxide as described in US Patent No. 4654267 followed by reaction with methacrylic acid to provide a terminal vinyl grouping. Solution copolymerisation with methacrylic acid yields a polymeric coating carrying terminal carboxyl groups as in R452 beads referred to below. Similarly, amino groups can be introduced by reacting a diamine with the above product of the reaction with a diepoxide as in the R240, R442 and R469 beads, while reaction with a hydroxylamine such as aminoglycerol introduces hydroxy groups as in the M450 and L255 beads.

Dynabeads M450 (diameter 4.5 microns) which may be obtained from Dynal, Oslo, Norway have been coated with a monomeric epoxide, resulting in a mixture of epoxy and hydroxy groups. Contact with water however, converts the epoxy groups to hydroxy groups.

Dynabeads M-280 (diameter 2.8 microns) are polystyrene beads having hydroxyl groups which have been converted into tosyloxy groups by reaction with p-toluene sulphonyl chloride.

Using functionalised coatings of the above types, we have found the non-specific binding of DNA and/or RNA to be very low, particularly in the case of the carboxylated beads.

As indicated above, the probe and RE linker are preferably attached to the magnetic particles via carboxyl groups, the DNA being firstly provided with a 5'-terminal amino group which can be made to form an amide bond with the carboxyl using a carbodiimide coupling agent. 5'- attachment of DNA can also be effected using hydroxylated magnetic particles activated with CNBr to react with 5'- amino DNA.

The 3'-attachment of the oligonucleotide DNA can also be effected by chemical synthesis. Here again, the very uniform nature of the monodisperse particles provides uniform reaction rates particularly suited to synthesis in an automated synthesiser such as the Gene Assembler (Pharmacia AS). The magnetic particle needs to be provided initially with a hydroxyl or protected hydroxyl group. Dynabeads M-280 of Dynal A/S are well suited to this purpose. If necessary, however, other surface functions such as carboxyl could be used to attach a linker carrying a hydroxyl group or alternatively a 3'-attached nucleotide.

5'-Attachment may be effected by coupling of 5'-amino-oligonucleotides to tosyl-activated magnetic particles. The latter may be produced by tosylation of hydroxylated magnetic particles such as Dynabeads M-280 of Dynal A/S. Displacement of the tosyloxy group leaves the 5'-amino group directly attached to the magnetic beads.

Since biotin labelled nucleotides are commercially available, the 3'- end of DNA fragments can be labelled using DNA polymerase and these may be conveniently bound to avidin or streptavidin attached to the magnetic particles e.g. via a hydroxy group. The biotin label may be attached to the nucleotide by a spacer arm, such as one or more $\varepsilon$-aminocaproic acid moieties, to minimize steric hindrance.

In general, the functionalisation of the beads and subsequent attachment of probes is advantageously such that each magnetic particle carries $10^3$-$10^6$ probes. (1-300 pmols per mg). The uniform size of the magnetic particles is of advantage in ensuring uniform probe density when the probes are reacted with the particles. Uniform probe density is important in ensuring that all the probes behave in substantially the same way in the various procedures in which they are used.

It is a remarkable feature of the magnetic particles that enzyme activity appears to take place very close to the particle surface e.g. within 7 bases. Thus, if an RE site is present in a linker sequence as discussed hereinafter and if the probe is subsequently used as a primer, it is found that sscDNA and hence ds cDNA can be synthesised by the DNA polymerase past the RE site towards the bead surface and can thus itself readily be cleaved by the appropriate endonuclease. In the case of the carboxylated beads, it is found that the microsurface of the beads is extremely irregular, presenting an unusually large surface area which may reduce steric hinderance to hybridisation and enzyme activity close to the surface. On the other hand the non-specific binding to such carboxylated beads is not increased.

According to a further feature of the invention we provide a kit for the detection and/or quantitative determination of target RNA or DNA comprising

(a) magnetic particles carrying single stranded 5'-attached DNA probe;
(b) a reverse transcriptase and/or a polymerase
(c) labelled nucleotides; and
(d) appropriate buffers.

The following examples are given by way of illustration only:-

Example 1(a)

Carbodiimide (EDC) mediated attachment of 5' -NH$_2$ probes to carboxyl beads.

(a) The reaction used for attaching probes to carboxyl beads is as follows. Amino groups introduced at the 5'-end of the probes using a one-step reaction method described by Chu et al. (Chu, B.C.F., and Orgel, L.E. (1985) DNA 4, 327-331.), results in a greater nucleophilicity of the terminal primary amino group of the alkyl linker as compared to the amino functionalities of the bases. It was therefore expected that the carboxyl groups on the beads would react preferentially with these primary amino groups.

100 ug 5'-NH$_2$ modified probe in 600 ul of 0.1 M imidazole-buffer pH 7, 0.1 M EDC were added per mg of R452 carboxyl beads. The reaction mixtures were incubated for 20 hours at room temperature with gentle shaking.

(b) NH$_2$ modified probes were made using Applied Biosystem synthesizer and Aminolink II.

The coupling reactions were as follows:

10 $\mu$g 5'NH$_2$ modified probe in 100 $\mu$l of 0.1M imidazole buffer pH 7.0, 0.1M EDC was added per mg of R452 carboxyl beads. The reaction mixtures were incubated for 20 hours at room temperature on a roller mixer (Coulter) followed by washing in TE buffer containing 0.1M NaCl (4x).

Hydridization efficiency:

A range of beads with different amount of probe attached were tested in hybridization experiments with a complementary 25 mer polydT probe.

The beads covered the range 1-250 pmol probe attached per mg beads.

Increasing amounts of 25 mer polydA oligonucleotide hybridized with increasing amounts of probe attached. 193 pmol hybridized to beads with 250 pmol attached. However, when the target molecule was in the range of 1000 bp (control mRNA Promega Corporation) there was no difference in hybridization efficiency between the bead with 100 pmol of attached probe compared with the more densely coupled beads.

## Example 2

Carbodiimide (EDC) mediated attachment of 5'-phosphate-probes to amino beads.

Probes were attached via a phosphoramidate linkage to 3 different amino beads by the method described by Ghosh et al. (Ghosh, S.S., and Musso, G.F. (1987) Nucl. Acids Res. 15, 5353 - 5372.). The amount of DNA attached to the different beads varied from 1.4-11.3 micrograms/mg.

The R469 beads which carry an amino group at the termini of an polyethylene glycol linker (8 atoms), bind a larger amount of probes than R240 beads which carry the amino group on a shorter linker (3 atoms). when the linker is made longer (number of atoms 20) as in the case of for the R442 beads, a decrease in the amount of probes bound to the beads is observed. This is probably due to secondary structures of the linkers which results in the terminal amino group becoming unavailable for coupling.

The amount of non-specifically bound DNA varies amoung the beads (7-30%) probably according to number of amino groups per unit of surface area. The R469 beads, which bind the largest amount of probes covalently (11 ug/mg), showed the lowest non-specific binding.

The acid lability of the phosphoramidate bond (Chu, B.C.F., Wahl, G.M., and Orgel, L.E. (1983) Nucl. Acids Res. 11, 6513 - 6529.) is used for measuring degree of end-attachment by acid hydrolysis. The amount end-attached probes varies between the different beads from 20-65%, and again, the R469 bead seems to be the preferable one with 65% of the probes end-attached.

We were able to attach twice as much probe material to the R469 beads by performing the reaction in imidazole buffer pH 7 for 3 hours at 50°C, instead of pH 6, for 24 hours at room temperature. An increase in molarity of EDC from 0.1 M to 0.2 M resulted in a 20% decrease in amount of probes on the R469 beads (data not shown).

### General Method

600 pmole (6 ug) of oligo A (36 mer) were dissolved in 1 ml of 0.1 M imidazole, pH 7, 0.1 M EDC and mixed with 5 mg of amino beads, and incubated for 3 hours at 50°C.

### Example 3

Coupling of 5'NH$_2$ probes to tosyl activated beads

NH$_2$ groups were introduced at the 5' end of oligonucleotides using Applied Biosystems DNA Synthesizer 381A and Aminolink II to introduce the primary NH$_2$ group at the 5' end. Aminolink II is supplied from Applied Biosystems. After synthesis these amino modified oligonucleotides were used directly in the coupling experiment.

Tosyl activated M-280 beads are commercially available from DYNAL AS, Oslo.

### Coupling procedure:

10 mg of tosyl activated beads were mixed with 50 µg NH$_2$ modified oligonucleotide in 100 µl 0.5M Na$_2$HPO$_4$ and incubated at 37°C for 20 hours on a roller mixer (Coulter) followed by washing in TE buffer containing 0.1M NaCl (4x).

### Example 4

Direct synthesis

Dynabeads R 488 beads were used. They are the same beads as M-280 except that the diameter is 3.15 microns instead of 2.8 microns and they contain primary OH groups on the surface as in the M-280 beads.

Using the synthesizer (Pharmacia Gene Assembler) the 3' end of DNA will be attached to the surface.

Only small modifications were necessary to fit the 3.15 micron beads. In the standard small scale column from applied Biosystems teflon filters with cut off at 3.0 microns were installed, the beads loaded and the column assembled.

Since this support does not contain dimethyltrityl (DMTr) groups and this machine stops if no such chemical is released in the first steps in the first cycle, small modifications in the start procedure were introduced. The synthesis was started using a standard ABI small scale column until the DMTr groups were released. Then the Gene Assembler was stopped manually and the modified column with magnetic beads was put into the Gene Assembler. The standard synthesis programme as recommended by the manufacturer was then followed. Deprotection was as recommended by Pharmacia. Direct synthesis was used to produce oligo(dT)$_{25}$ and the following sequence from the C region of the kappa light chain gene:

$$5\,'-\text{TCACTGGATGGTGGGAAGATGGATACAGTTGGTGCA}-3\,'.$$

## Example 5

## Materials and methods

## Magnetic beads

Dynabeads M-280 Streptavidin (Dynal A.S, Box 158, N-0212 Oslo) were used as solid phase. These are monodisperse superparamagnetic polymer particles with a diameter of 2.8 μm covalently coupled with Streptavidin. They have a surface area of 4.3 m²/g.

## Biotin binding capacity

100 μl 6 x SSPE (Standard saline with phosphate and EDTA: Maniatis) containing 1 nmol $^{14}$C-Biotin (Amersham) was added to 0.5 mg beads (prewashed in 6 x SSPE) and placed on a roller mixer (Coulter) at room temperature for 15 minutes.

After two separate washes in 6 x SSPE the fraction of bound $^{14}$C-Biotin was measured by scintillation counting.

## Deoxyoligonucleotides

Deoxyoligonucleotides were synthesized on an Applied Biosystems 381A DNA synthesizer.

Chemicals were purchased from Applied Biosystems. 5'amino modified deoxyoligonucleotides were made using AminolinkII.

The immunoglobulin light kappa chain probe used was:

$$5\,'-\text{TCACTGGATGGTGGGAAGATGGATACAGTTGGTGCA}-3\,'.$$

## Biotinylation of probes

Biotin XNHS ester (Clontec N-succinimidyl of N-biotinyl ε-caproic acid) was used as recommended by the supplier.

0.1 μmol of NH$_2$-modified oligo(dT)$_{25}$ in 90 μl of water was added 10 μl labelling buffer (1M sodium bicarbonate/carbonate, pH 9.0) and vortexed.

Finally 25 μl Biotin XNHS ester (100 mg/ml) in dimethylformamide was added and incubated at room temperature overnight.

Excess labelling reagent and buffer was removed in a Sephadex G50 spin column.

The 5'Biotin oligo(dT)$_{25}$ was endlabelled using the fill in reaction by Klenow polymerase, $\alpha$-[$^{32}$P]-dTTP and oligo(dA)$_{25}$ as template. Excess label was removed using a Sephadex G50 spin column.

## Preparation of oligo(dT) Dynabeads (T-beads)

200 μg Biotinylated oligo(dT)$_{25}$ (24 n mol) in 2.5 ml 6 x SSPE was mixed with 50 mg prewashed Dynabeads M-280 Streptavidin and incubated on a roller mixer for 15 minutes at room temperature.

After two washes in 6 x SSPE the beads were stored at 4°C in 6 x TE, 0.1 % SDS.

## Oligonucleotide hybridization assay

In the standard assay to measure hybridization capacity of different batches of T-beads, 0.1 mg of the beads in an eppendorf tube was washed once with 6 x SSPE, 0.1 % SDS. A magnet rack (MPC-E, Dynal A.S., Oslo) was used to aggregate beads between each step.

After removal of the washing buffer, 50 μl hybridization solution (6 x SSPE, 0. 1% SDS), containing 50 pmol of oligo(dA)$_{25}$ with trace amount (1-2 x 10$^5$ cpm) α- [$^{32}$P] -dATP-labelled oligo(dA)$_{25}$ was added.

After gentle mixing the tube was left to hybridize for two minutes at room temperature.

The hybridized beads were washed twice with 2 x SSPE, 0.1 % SDS at room temperature and the percentage of oligo(dA)$_{25}$ hybridized to the oligo(dT)$_{25}$ Dynabeads was measured in a scintillation counter.

## Labelling of poly A mRNA tracer

1 μg 1200 bp mRNA with a 3'polyA$_{30}$ tail (Promega) was mixed with 2.5 pool oligo(dT)$_{25}$ in 10 μl 5 x Klenow buffer, 1 u RNasin (Promega), 10mM DDT. After two minutes at room temperature 10 μCi α-[$^{32}$P]-dATP, 1 u Klenow polymerase (Amersham) and water up to 50 μl were added and incubation continued for 60 minutes at 15°C. Excess α-[$^{32}$P]-dATP was removed using a Sephadex spin column.

## Buffers for poly(A) mRNA hybridization to Dynabeads M-280 Streptavidin coupled with oligo(dT)$_{25}$

Poly(A) binding buffer:
0.5M LiCl, 10 mM Tris-Cl, pH 7.5, 1 mM EDTA, 0.1 % sodium dodecylsulphate.
Middle wash buffer:
0.15M LiCl, 10 mM Tris-Cl, pH 7.5, 1 mM EDTA, 0.1 % sodium dodecylsulphate.
Elution buffer: 2 mM EDTA, 0.1 % SDS. Depending on the subsequent use of the purified mRNA SDS may be omitted in the last washing step and in the elution buffer.

## Example 6

### Quantitative assay for polyadenylated mRNA present in a solution

mRNA from a hybridoma cell line AB$_4$ has been isolated by the use of magnetic beads but a method more sensitive than UV-spectrophotometry was needed to measure its concentration.

Commercially available polyA mRNA from mouse pancreas (clontech) of known concentration was suspended into the following dilutions. 50 μl 6 x SSPE containing 2 units of RNasin and 10 mM DDT, and respectively 100 ng (0.25 pmols), 50 ng (0.12 pmols), 20 ng (50 fmols) and 10 ng (25 fmols). [The μg/mol correlation is calculated by assuming an average length of the polyA mRNA to be 1200 bases. This gives a molecular weight of 396.000 daltons and suggests that 1 μg mRNA is 2.5 pmols.]

Four similar dilutions were made from the AB$_4$ -mRNA but of unknown concentration.

The DNA probe named RET was coupled to magnetic beads (R502) and gave a hybridization capacity of 8 pmols/mg beads of a (dA)$_{17}$oligo-nucleotide.

0.5 mg of RET beads were added to each of the eight mixtures and the mRNA was let to hybridize to the magnetic probes for 10 minutes at room temperature. It was known from earlier experiments that more than 90% of the mRNA is hybridized in this time when such a large molar excess of probes, compared to mRNA, is used (in this case greater than 20:1).

The beads were collected (aggregated) and washed once in 200 μl 2 x SSPE at room temperature.

To each of the eight tubes, together with a negative control of 0.5 mg RET-bead without mRNA, was added 50 μl of a larger premixed reaction cocktail. The 50 μl mixture contains:
1 Klenow buffer (50 mM NaCl, 20 mM Tris-HCl pH 7.5)
1mM EDTA
1 u Klenow Enzyme
1 μCi α-[$^{32}$P]-dTTP
100 pmols cold dTTP

The tubes were incubated at 15°C for 60 minutes on a roller machine to avoid sedimentation of beads. After incubation the beads were washed twice in 200 μl 0.2 M NaOH, 0.5 M NaCl and resuspended in 50 μl TE-buffer (10 mM Tris-HCl, pH 7.4, 1 mM EDTA).

The amount of incorporated radioactivity was measured in a scintillation counter.

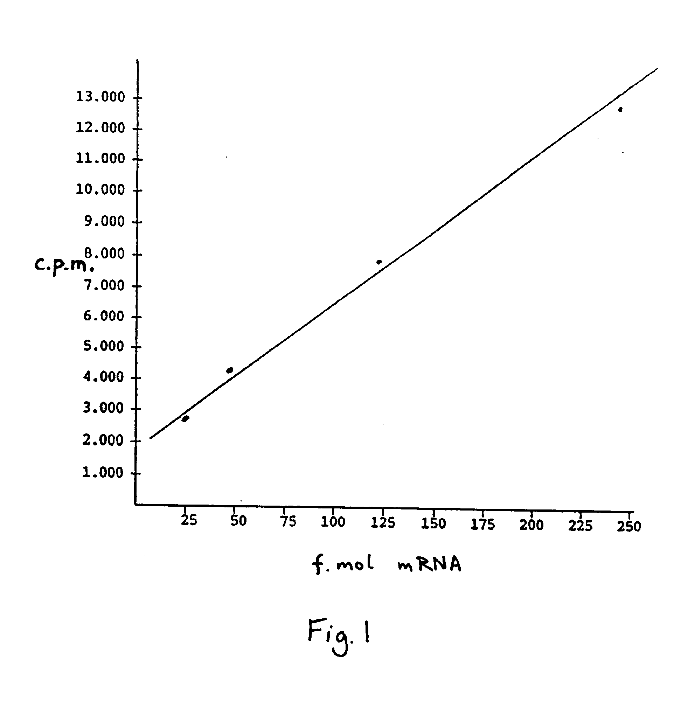

The results (as shown in Fig. 1) gave a clear linear correlation between added standard mRNA and incorporated dTTP. Two of the test samples (AB$_4$ were within the range of selected standard dilutions and each of them could be used to decide the actual AB$_4$ mRNA concentration from the standard curve in Fig. 1.

The negative control gave zero incorporation of dTTP as expected since no template was available.

Example 7

A similar set of experiments as in Example 6 but with 100 μg Dynabeads M280 streptavidin particles coupled with biotinylated oligod$T_{25}$ were allowed to hybridize to 100 ng, 50 ng, 20 mg and 10 ng of Promega control mRNA (~1.2 kb kanamycin mRNA containing a 30A residue polyA tail) and after two washing steps in 2 x SSPE a standard single strand cDNA synthesis (Promega protocol) was performed using reverse transcriptase and ($\alpha^{32}$p)dCTP after two washes in 0.2 M NaOH, and resuspension in 50 μl TE (100mM tris pH7.5, 10mM EDTA). The amount of incorporated radioactivity was measured in a scintillation counter with similar result as in Example 6.

## Claims

1. A method for the detection and/or quantitative determination of target RNA or DNA in an analyte sample which includes the steps of:
   a) contacting the analyte with magnetic particles carrying a single stranded 5'-attached DNA probe capable of binding to said RNA or DNA;
   b) contacting the magnetic particles with an aqueous solution of a reverse transcriptase or polymerase in the presence of labelled nucleotides using the probe as a primer whereby labelled complementary single stranded DNA is formed if said RNA or DNA was present in said analyte; magnetically aggregating the magnetic particles onto a surface and removing said solution; and
   c) detecting the presence or absence of said label and/or determining the amount of said label.

2. A method as claimed in claim 1 wherein the chain length of complementary single stranded DNA is limited.

3. A method as claimed in claim 2 wherein a predetermined amount of dideoxynucleotides are present at step (b) to limit chain synthesis to a predetermined average length thereby enabling the total amount of label to be correlated with the number of target nucleic acid molecules.

4. A method as claimed in any one of claims 1, 2 or 3 wherein the probe used in step (a) is oligo-dT.

5. A method as claimed in claim 2 wherein the probe binds to said DNA or RNA a predetermined distance from a chain terminating point of said DNA or RNA to enable the total amount of label to be correlated with the number of target nucleic acid molecules.

6. A method as claimed in any preceding claim wherein polymerase chain reactions (PCR) occurs between steps (a) and (b) to amplify said target RNA or DNA, primers for PCR being 5'-attached to magnetic particles.

7. A method according to any preceding claim wherein washing steps occur between each of steps (a), (b) and (c).

8. A method according to any preceding claim wherein the labelled nucleotides comprise a label selected from the group consisting of radiolabels, fluorophores, chromophores, and a low molecular weight ligand of a specific binding pair.

9. A method of detecting the presence of and/or amount of a microorganism in a sample wherein RNA or DNA of said microorganism is detected according to any method as claimed in any one of claims 1 to 8.

10. A kit for the detection and/or quantitative determination of target RNA or DNA comprising
    (a) magnetic particles carrying single stranded 5'-attached DNA probe ;
    (b) a reverse transcriptase and/or a polymerase;
    (c) labelled nucleotides ; and
    (d) appropriate buffers.

## Patentansprüche

1. Ein Verfahren zum Nachweis und/oder zur quantitativen Bestimmung von Target-RNA oder -DNA in einer Analysenprobe, welches die folgenden Stufen umfasst:

a) Zusammenbringen der Analysenprobe mit magnetischen Parti-keln, die eine einzelsträngige, 5'-gebundene DNA-Probe tragen, die imstande ist, an diese RNA oder DNA zu binden;

b) Zusammenbringen der magnetischen Partikel mit einer wäss-rigen Lösung einer reversen Transkriptase oder Polymerase in Gegenwart von markierten Nukleotiden unter Verwendung der Probe als Primer, wobei markierte komplementäre einzelsträngige DNA gebildet wird, falls diese RNA oder DNA in dieser Analysenprobe vorhanden war; magnetisches Binden der magnetischen Partikel an eine Oberflächeund Entfernen dieser Lösung; und

c) Nachweisen der Anwesenheit oder der Abwesenheit dieser Markierung und/oder Bestimmen der Menge dieser Markierung.

2. Ein Verfahren gemäss Anspruch 1, worin die Kettenlänge der komplementären einzelsträngigen DNA begrenzt ist.

3. Ein Verfahren gemäss Anspruch 2, worin in der Stufe b) eine vorbestimmte Menge an Didesoxynukleotiden vorhanden ist, um die Kettensynthese auf eine vorbestimmte mittlere Länge zu begren-zen, um dadurch zu ermöglichen, die Gesamtmenge der Markierung auf die Nummer der Target-Nukleinsäuremoleküle zu beziehen.

4. Ein Verfahren gemäss einem der Ansprüche 1, 2 oder 3, worin die in Stufe (a) verwendete Probe Oligo-dT ist.

5. Ein Verfahren gemäss Anspruch 2, worin die Probe an diese DNA oder RNA in einer vorbestimmten Entfernung von einem Ketten-Endpunkt dieser DNA oder RNA bindet, um zu ermöglichen, die Gesamtmenge der Markierung auf die Anzahl der Target-Nukleinsäuremoleküle zu beziehen.

6. Ein Verfahren gemäss einem der vorhergehenden Ansprüche, worin Polymerase-Kettenreaktionen (PCR) zwischen den Stufen (a) und (b) erfolgen, um diese Target-RNA oder -DNA zu amplifi-zieren, wobei Primer für die PCR an die magnetischen Partikel 5'-gebunden sind.

7. Ein Verfahren gemäss einem der vorhergehenden Ansprüche, worin zwischen jeder der Stufen (a), (b) und (c) Waschstufen erfolgen.

8. Ein Verfahren gemäss einem der vorhergehenden Ansprüche, worin die markierten Nukleotide eine Markierung enthalten, die aus der Gruppe bestehend aus Radiomarkierungen, Fluorophoren, Chromophoren, und einem Liganden von niedrigem Molekulargewicht eines spezifischen Bindungspaares ausgewählt ist.

9. Ein Verfahren zum Nachweis der Anwesenheit von und/oder der Bestimmung der Menge eines Mikroorganismus in einer Probe, worin RNA oder DNA dieses Mikroorganismus durch eines der in einem der Ansprüche 1 bis 8 beanspruchten Verfahren nachge-wiesen wird.

10. Ein Kit zum Nachweis und/oder quantitativen Bestimmung von Target-RNA oder -DNA umfassend

(a) magnetische Partikel, die eine einzelsträngige, 5'-gebundene DNA-Probe tragen;

(b) eine reverse Transkriptase und/oder Polymerase;

(c) markierte Nukleotide; und

(d) geeignete Puffer.

**Revendications**

1. Procédé pour la détection et/ou la détermination quantitative d'ARN ou d'ADN cible dans un échantillon d'une substance à analyser, qui comprend les étapes de :

a) contact de la substance à analyser avec des particules magnétiques portant une sonde d'ADN mo-monocaténaire attachée en 5' capable de liaison audit ARN ou ADN ;

b) contact des particules magnétiques avec une solution aqueuse d'une transcriptase réverse ou d'une polymérase en présence de nucléotides marqués, en utilisant la sonde comme amorce pour qu'il se forme de l'ADN monocaténaire complémentaire marqué si ledit ARN ou ADN est présent dans ladite substance à analyser ; agrégation magnétique des particules magnétiques sur une surface et élimination de ladite solution ; et

c) détection de la présence ou de l'absence dudit marqueur et/ou détermination de la quantité dudit marqueur.

2. Procédé selon la revendication 1, dans lequel la longueur de la chaîne de l'ADN monocaténaire complémentaire est limitée.

3. Procédé selon la revendication 2, dans lequel une quantité prédéterminée de didésoxynucléotides est présente dans l'étape (b) pour limiter la synthèse de chaîne à une longueur moyenne prédéterminée, afin de pouvoir établir une corrélation entre la quantité totale du marqueur et le nombre des molécules d'acide nucléique cible.

4. Procédé selon l'une quelconque des revendications 1, 2 ou 3, dans lequel la sonde utilisée dans l'étape (a) est un oligo-dT.

5. Procédé selon la revendication 2, dans lequel la sonde se lie audit ADN ou ARN à une distance prédéterminée d'un point de terminaison de chaîne dudit ADN ou ARN, pour permettre d'établir une corrélation entre la quantité totale de marqueur et le nombre des molécules d'acide nucléique cible.

6. Procédé selon l'une quelconque des revendications précédentes, dans lequel des réactions d'amplification en chaîne par polymérase (PCR) se produisent entre les étapes (a) et (b) pour amplifier ledit ARN ou ADN cible, des amorces de PCR étant attachées en 5' aux particules magnétiques.

7. Procédé selon l'une quelconque des revendications précédentes, dans lequel des étapes de lavage ont lieu entre chacune des étapes (a), (b) et (c).

8. Procédé selon l'une quelconque des revendications précédentes, dans lequel les nucléotides marquées comprennent un marqueur choisi dans le groupe constitué par les radiomarqueurs, les fluorophores, les chromophores et un ligand de bas poids moléculaire d'une paire à liaison spécifique.

9. Procédé de détection de la présence et/ou de la quantité d'un microorganisme dans un échantillon, dans lequel on détecte l'ARN ou l'ADN dudit microorganisme selon l'un quelconque des procédés revendiqués dans l'une quelconque des revendications 1 à 8.

10. Nécessaire pour la détection et/ou la détermination quantitative d'ARN ou d'ADN cible comprenant :

(a) des particules magnétiques portant une sonde d'ADN monocaténaire attachée en 5' ;

(b) une transcriptase réverse et/ou une polymérase ;

(c) des nucléotides marquées ; et

(d) des tampons appropriés.

Fig. 1